# EUROPEAN PATENT APPLICATION

(11) **EP 4 631 528 A1**
(43) Date of publication of application: **15.10.2025**
(21) Application number: 23901156.2
(22) Date of filing: 08.12.2023
(51) Int. Cl.: A61K 48/00, A61K 47/69, A61K 9/51, A61K 31/713, A61P 35/00, A61P 29/00, A61P 31/12

(54) **NANOPARTICLE COMPRISING PEPTIDE-BASED CONJUGATE FOR DELIVERING OLIGONUCLEOTIDE INTO TARGET CELL AND PHARMACEUTICAL COMPOSITION COMPRISING SAME**

(30) Priority: 09.12.2022 KR 20220171767
(71) Applicant: Nibec Co., Ltd., Chungcheongbuk-do 27816 (KR); Seoul National University R&DB Foundation, Seoul 08826 (KR)
(72) Inventor: PARK, Yoon Jeong, Seoul 08291 (KR); CHUNG, Chong-Pyoung, Seoul 05618 (KR); LEE, Jue-Yeon, Gwacheon-si, Gyeonggi-do 13831 (KR)
(74) Representative: Mewburn Ellis LLP
(86) International application number: PCT/KR2023/020217
(87) International publication number: WO 2024/123139

(57) **Abstract**

The present invention relates to: a carrier for delivering oligonucleotides intended for therapeutic use into cells, wherein, to inhibit a target protein or promote expression of a target protein in a cell, a peptide-based conjugate is prepared, and a nanoparticle consisting of a peptide-based conjugate comprising oligonucleotides; and a pharmaceutical composition comprising the carrier. It was confirmed that, by using the nanoparticle consisting of the peptide-based conjugate according to the present invention, oligonucleotides were effectively delivered into cells. In addition, by confirming that the expression of a cancer-causing protein is reduced by oligonucleotides and that an anticancer effect is exhibited in an animal model in which cancer has occurred, the nanoparticle consisting of the peptide-based conjugate was found to be effective in the delivery of oligonucleotides.

## Description

### Technical Field

The present invention relates to a nanoparticle comprising a peptide-based conjugate loaded with an oligonucleotide for inhibiting a target protein in a cell or promoting target protein expression in a cell, and a pharmaceutical composition comprising the same.

### Background Art

Oligonucleotides have been developed as therapeutic agents for controlling genes that cause cancer and infectious diseases, especially in the form of siRNA and mRNA. Recently, as an mRNA vaccine encoding the spike protein of the COVID-19 virus in the form of mRNA, which induces an immune response upon injection thereof, was approved by the FDA, the application of mRNA has attracted attention. mRNA is nontoxic because it is mRNA synthesized *in vivo.* However, siRNA and mRNA are rapidly degraded by nucleases *in vivo,* are negatively charged, and cannot target specific lesions. In addition, siRNA and mRNA are effective only when they act within the cytoplasm, but they cannot penetrate the cell membrane by themselves due to their negative charge and large size. Therefore, in order to overcome this limitation, carriers capable of stably delivering oligonucleotides into cells are required.

The most widely used carriers are lipid nanoparticles, and oligonucleotide carriers using various cationic lipids are known (US 2006/0083780, US 2006/0240554, US 2008/0020058, US 2009/0263407, US 2009/0285881, WO 2009/086558, WO 2009/127060, WO 2009/132131, WO 2010/042877, WO 2010/054384, WO 2010/054401, WO 2010/054405, WO 2010/054406, and WO 2010/105209). Conventional cationic lipids such as CLinDMA and DLinDMA have been used for oligonucleotide delivery to the liver, but are known to suffer from non-optimal delivery efficiency along with liver toxicity at high doses.

Lipid nanoparticles (LNPs) are produced using cationic lipids, cholesterol, and polyethylene glycol (PEG). Cationic lipids allow oligonucleotides to pass through the cell membrane, cholesterol allows oligonucleotides to retain the shape thereof, and PEG allows for long circulation of lipid nanoparticles. However, there are reports that PEG induces anaphylactic reactions by generating antibodies against PEG. Therefore, there is a need for new oligonucleotide delivery technology capable of safely and efficiently delivering oligonucleotides into cells.

Accordingly, the present inventors have produced a peptide-based conjugate using peptides comprising a sequence for target-cell surface recognition, a sequence having cell-penetrating function, a sequence that facilitates endosomal escape, and a sequence capable of binding to an oligonucleotide, and a lipid capable of protecting the oligonucleotide, in order to produce a nanoparticle capable of safely and effectively delivering the oligonucleotide into a cell, and have found that, when the nanoparticle comprising the peptide-based conjugate is used, the oligonucleotide is effectively delivered into a cell, and that the loaded oligonucleotide reduces the expression of a cancer-causing protein and exhibits an anticancer effect in an animal model of cancer, thereby completing the present invention.

The above information disclosed in this Background section is only for enhancement of understanding of the background of the present invention. Therefore, it may not contain information that forms a conventional art that is already known in the art to which the present invention pertains.

### Summary of the Invention

An object of the present invention is to provide a peptide-based conjugate capable of safely and effectively delivering an oligonucleotide into a cell.

Still another object of the present invention is to provide a nanoparticle for intracellular delivery of an oligonucleotide, in which the peptide-based conjugate and the oligonucleotide are bound to each other, and a composition for intracellular delivery of an oligonucleotide comprising the nanoparticle.

Yet another object of the present invention is to provide a pharmaceutical composition for preventing or treating a disease comprising the nanoparticle.

Still yet object of the present invention is to provide a method for preventing or treating a disease comprising administering the nanoparticle.

A further object of the present invention is to provide the use of the nanoparticle for preventing or treating a disease.

Another further object of the present invention is to provide the use of the nanoparticle for the manufacture of a medicament for the prevention or treatment of a disease.

In order to achieve the above objects, the present invention provides a peptide-based conjugate having Structural Formula 1 below as a basic unit:

[Structural Formula 1] A-B-D-E

wherein A is an RNA-binding peptide, B is a fatty acid having 12 to 20 carbon atoms, a cationic lipid, an amphipathic polypeptide, or an amphipathic polymer, D is a peptide having a cell-penetrating function, and E is a peptide that recognizes a target cell surface.

The present invention also provides a nanoparticle in which the peptide-based conjugate and an oligonucleotide are bound to each other.

The present invention also provides a composition for intracellular delivery of an oligonucleotide comprising a nanoparticle in which the peptide-based conjugate and the oligonucleotide are bound to each other.

The present invention also provides a pharmaceutical composition for preventing or treating a disease comprising the nanoparticle in which the peptide-based conjugate and an oligonucleotide are bound to each other.

The present invention also provides a method for preventing or treating a disease comprising administering the nanoparticle.

The present invention also provides the use of the nanoparticle for preventing or treating a disease.

The present invention also provides the use of the nanoparticle for the manufacture of a medicament for the prevention or treatment of a disease.

### Brief Description of Drawings

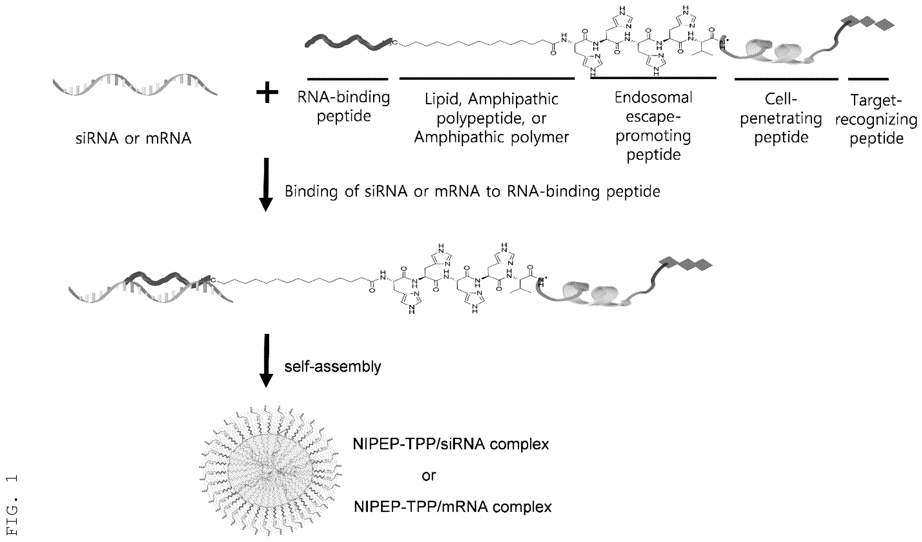

FIG. 1 shows a process of forming a nanoparticle by self-assembly after an oligonucleotide is bound to an RNA-binding peptide of a conjugate comprising the RNA-binding peptide, a lipid, a peptide that facilitates endosomal escape, a peptide having a cell-penetrating function, and a target-recognizing peptide.
FIG. 2A is a TEM image showing nanoparticles comprising mRNA and a peptide-based conjugate, and FIG. 2B is a TEM image showing nanoparticles comprising siRNA and a peptide-based conjugate.
FIG. 3 shows the results of analyzing the expression of KRAS degradation protein in cells by Western blot analysis after treating the cells with nanoparticles formed by a peptide-based conjugate and mRNA encoding the KRAS degradation protein.
FIG. 4 shows the results of analyzing the expression of firefly luciferase *in vivo* by IVIS after injecting nanoparticles formed by a peptide-based conjugate and mRNA encoding firefly luciferase into mice.

### Detailed Description and Preferred Embodiments of the Invention

Unless otherwise defined, all technical and scientific terms used in the present specification have the same meanings as commonly understood by those skilled in the art to which the present invention pertains. In general, the nomenclature used in the present specification is well known and commonly used in the art.

In the present invention, nanoparticles comprising a peptide-based conjugate capable of safely and efficiently delivering a functional oligonucleotide into cells were produced. First, a peptide-based conjugate was produced using peptides comprising a sequence for target cell surface recognition, a sequence having a cell-penetrating function, an endosomal escape-promoting sequence, and a sequence capable of binding to an oligonucleotide, and a lipid capable of protecting the oligonucleotide, and then nanoparticles were produced by binding an oligonucleotide to the peptide-based conjugate. It was found that, when the nanoparticles comprising the peptide-based conjugate were used, the oligonucleotide was effectively delivered into cells, and the loaded oligonucleotide reduced the expression of a cancer-causing protein and exhibited an anticancer effect in an animal model of cancer.

Therefore, in one aspect, the present invention relates to a peptide-based conjugate having Structural Formula 1 below as a basic unit:

[Structural Formula 1] A-B-D-E

wherein A is an RNA-binding peptide,
B is a fatty acid having 12 to 20 carbon atoms, a cationic lipid, an amphipathic polypeptide, or an amphipathic polymer,
D is a peptide having a cell-penetrating function, and
E is a peptide that recognizes a target cell surface.

In the present invention, A may comprise a peptide sequence to which RNA binds. A may be a peptide represented by any one of the amino acid sequences of SEQ ID NO: 1 to SEQ ID NO: 15, without being limited thereto.
SEQ ID NO: 1: LKKLLKLLKKLLKLAG
SEQ ID NO: 2: IKKLIKIIKKLIKLAG
SEQ ID NO: 3: LRRLLRLLRRLLRLAG
SEQ ID NO: 4: LKKLLKLLOrnKLLDprLAG
SEQ ID NO: 5: LRRLLRLLOrnRLLDprLAG
SEQ ID NO: 6: LRKIIRLIOrnKLLDprLAG
SEQ ID NO: 7: LKKLLKLLOrnKLLKLAG
SEQ ID NO: 8: WKKLLKLLKKLLKLAG
SEQ ID NO: 9: WRRLLRLLRRLLRLAG
SEQ ID NO: 10: WRKLLRLLKKLLKLAG
SEQ ID NO: 11: LKKLLDbuLLKKLLKWAG
SEQ ID NO: 12: LRRLLDbuLLRRLLRWAG
SEQ ID NO: 13: LKRLIDbuIIKKLIKWAG
SEQ ID NO: 14: LKKLLKWLOrnKLLDprLAG
SEQ ID NO: 15: LRRLLRWLOrnRLLDbuLAG

Orn = Ornithine, Dbu = 2,4-diaminobutyric acid, Dpr = 2,3-diaminopropionic acid.

In the present invention, A may be, without limitation, any peptide to which RNA may bind. For example, A may be a nucleic acid-binding peptide disclosed in US 2020/0207834 A1, etc., without being limited thereto.

In the present invention, B may be a fatty acid having 12 to 20 carbon atoms, a cationic lipid, an amphipathic polypeptide, or an amphipathic polymer.

The fatty acid may be a saturated fatty acid or unsaturated fatty acid having 12 to 20 carbon atoms.

In the present invention, examples of the cationic lipid include, but are not limited to, 3β-[N-(N',N'-dimethylaminoethane carbamoyl cholesterol (DC-Chol); 1,2-dioleoyl-3-trimethylammonium-propane (DOTAP); 1,2-dioleoyl-3-dimethylammonium-propane (DODAP); dimethyldioctadecylammonium bromide (DDAB); 1,2-dilauroyl-sn-glycero-3-ethylphosphocholine chloride (DL-EPC); N-[1-(2,3-dioleyloxy) propyl] -N-N-N-trimethylammonium chloride (DOTMA); N-[1-(2,3-dioleyloxy) propyl]-N-N-N-dimethylammonium chloride (DODMA); 1,2-dilauroyl-sn-glycero-3-ethylphosphocholine chloride (DOTMA); N,N-dioctadecyl-N,N-dimethylammonium chloride (DODAC); N-(1-(2,3-dioleyloxy)propyl)-N-2-(sperminecarboxamido)ethyl)-N,N-dimethylammonium trifluoroacetate (DOSPA); 1,2-dimyristyl oxypropyl-3-dimethylhydroxyethylammonium bromide (DMRIE); dioctadecyl amido glycyl spermine (DOGS); neutral lipids conjugated to cationic modifiers; and combinations thereof. Further, a number of cationic lipids may be used in commercially available formulations, such as, Lipofectin (GIBCO), Lipofectamine (GIBCO), and Transfectam (Promega).

In the present invention, the term "amphipathic" means comprising hydrophilic and hydrophobic domains.

The amphipathic polypeptide refers to a polypeptide which comprises polar amino acids and non-polar amino acids and in which the polar amino acids and nonpolar amino acids are partially densely packed together to form a polar region and a nonpolar region. The polar amino acids include cysteine, glutamine, threonine, tyrosine, serine, or asparagine, and the nonpolar amino acids include phenylalanine, tryptophan, methionine, proline, valine, isoleucine, leucine, glycine, or alanine, without being limited thereto.

In the present invention, the amphipathic polypeptide may be in a form in which polar amino acids and nonpolar amino acids are densely packed together in the amino acid sequence, or may be in a form in which polar amino acids and nonpolar amino acids are not densely packed together in the amino acid sequence, but when a peptide comprising the amino acids forms a three-dimensional structure, the polar amino acids and nonpolar amino acids may be densely packed together by the three-dimensional structure, without being limited thereto. For example, the amphipathic polypeptide may be a combination of a plurality of identical or non-identical polar amino acids and a plurality of identical or non-identical non-polar amino acids.

In one embodiment of the present invention, the amphipathic polypeptide may be a peptide represented by the amino acid sequence of SEQ ID NO: 16, without being limited thereto.
SEQ ID NO: 16: MMIVRSQN

The amphipathic polymer is a polymer that is a combination of a hydrophobic polymer and a hydrophilic polymer, and is a polymer already known to those skilled in the art. The amphipathic polymer may include any one of poloxamer, which is a combination of hydrophilic and hydrophobic polymers, and a poly(ethylene oxide) (PEO)-polylactic acid (PLA) copolymer. The amphipathic polymer is preferably a poly(ethylene oxide) (PEO)-polylactic acid (PLA) copolymer, without being limited thereto and may be a self-assembling amphipathic polymer selected according to the intended use. In one embodiment of the present invention, the amphipathic polymer may be poloxamer, without being limited thereto.

In the present invention, D may comprise a peptide sequence having a cell-penetrating function. The peptide having cell penetrating function is not particularly limited as long as it has the property of entering a cell by a cell internalization (endocytosis) mechanism, but is preferably selected from the group consisting of cell-penetrating peptides or variants thereof disclosed in Korean Patent No. 10-0951719.

In one embodiment of the present invention, D may be a peptide selected from the group consisting of 4 to 12 arginines, 4 to 12 lysines, and 2 cysteines, or a combination thereof, without being limited thereto, and peptides or peptide analogues other than the above-mentioned peptides may also be used as long as they can penetrate the cell membrane.

In the present invention, E may comprise a peptide sequence that recognizes a target cell surface.

The target cell refers to a cell targeted by mRNA that binds to the RNA-binding peptide and is to be delivered into the cell. For example, when the oligonucleotide is mRNA, E may be a peptide that recognizes an immune cell surface, and the immune cell may be, but is not limited to, a B cell or a T cell.

In the present invention, E is a sequence that binds to the surface of existing target cells and may be newly discovered and applied through a phage display technique.

In one embodiment of the present invention, E may be a peptide represented by the amino acid sequence of SEQ ID NO: 17 that targets cancer cells, without being limited thereto.
SEQ ID NO: 17: CAIYPRH

In the present invention, the peptide-based conjugate may further comprise C, a peptide that facilitates endosomal escape, between B and D.

C may comprise a peptide sequence that facilitates endosomal escape, and is not particularly limited as long as it facilitates endosomal escape of the conjugate. Preferably, C may be selected from the group consisting of endosome-disrupting peptides or variants thereof disclosed in US 2020/0207834 A1.

In one embodiment of the present invention, C may be a peptide composed of 4 to 12 histidines, without being limited thereto.

In the present invention, A-B or B-C in Structural Formula 1 above may be linked by an amide bond, and the amide bond between A-B or B-C may be formed by a chemical synthesis method.

In the present invention, C-D-E may be produced by a chemical synthesis technique or a recombinant expression technique.

In one embodiment of the present invention, A and C-D-E may be synthesized by a solid-phase peptide synthesis method, and then B may be chemically bound thereto via an amide bond.

In another aspect, the present invention relates to a nanoparticle in which the peptide-based conjugate and the oligonucleotide are bound to each other.

In the present invention, the oligonucleotide may be DNA, siRNA, miRNA or mRNA, and preferably has the function of inhibiting or increasing the expression of a target gene, without being limited thereto.

In the present invention, the siRNA may have a nucleotide sequence complementary to a nucleotide sequence encoding a tumor-causing protein or a disease-causing protein, without being limited thereto.

In one example of the present invention, mRNA against mutant KRAS was used to inhibit the expression of mutant KRAS. The mRNA sequence encoding a protein that degrades KRAS may use mRNA having the base sequences of SEQ ID NO: 18 and SEQ ID NO: 19, but is not limited thereto. The mRNA sequence encoding a protein that degrades KRAS may use mRNA having the nucleotide sequences of SEQ ID NO: 18 and SEQ ID NO: 19, without being limited thereto.
SEQ ID NO: 18: mRNA sequence 1 encoding protein that degrades KRAS 
5′ cap-5′UTR-
AUGGAAGUCCAAUUGUUGGAAUCUGGGGGUGGGCUUGUCCAACCUGGUGGUUCACU
CCGUCUGAGCUGCGCCGCUUCCGGAUUCACUUUCUCCACCUUUUCUAUGAAUUGGGUUCGC
CAGGCCCCAGGCAAGGGACUUGAGUGGGUUAGCUAUAUCAGCAGGACCAGUAAAACCAUCU
ACUACGCCGACAGCGUUAAGGGAAGGUUCACUAUAUCCCGGGACAAUUCUAAGAAUACUUU
 
GUACUUGCAAAUGAACUCUUUGCGGGCAGAAGAUACUGCUGUCUACUAUUGUGCACGAGGU

AGGUUUUUUGAUUAUUGGGGUCAGGGGACCCUCGUAACCGUUUCCAGUGGGGGCAGCGAGG

GUAAGAGCUCCGGUUCAGGGUCAGAAUCUAAAAGCACUGGUGGCUCCGAUAUACAAAUGAC

CCAGAGUCCAAGCUCUCUUUCAGCCAGCGUGGGAGACAGGGUGACCAUCACCUGUCGAGCA

UCUCAAUCCAUUUCAUCAUACCUCAAUUGGUAUCAACAGAAGCCUGGGGAAGCACCAAAGU

UGCUUAUUUAUUCUGCCAGCGUACUGCAAAGUGGCGUACCUUCCCGCUUCUCCGGGUCUGG

GAGUGGCACUGAUUUUACCCUCACAAUUAGCAGCCUGCAACCCGAGGACUUCGCUACAUAC

UACUGCCAACAAAGUGUAAUGAUACCCAUGACAUUCGGGCAAGGCACAAAAGUCGAGAUAA

AGGGAAGUGGAGGAGGUGGCUCUAUGCCCCGCCGCGCUGAGAACUGGGAUGAGGCCGAAGU

UGGUGCUGAAGAGGCCGGAGUUGAAGAAUAUGGGCCAGAAGAAGAUGGCGGUGAGGAAAGC

GGGGCUGAAGAGAGCGGUCCAGAGGAGUCUGGUCCAGAGGAACUUGGAGCUGAGGAGGAGA

UGGAGGCCGGACGUCCACGUCCUGUCCUGCGAUCAGUUAACUCAAGGGAGCCCUCACAGGU

UAUCUUUUGUAAUCGAAGCCCUCGCGUGGUUUUGCCCGUCUGGCUCAACUUCGAUGGUGAA

CCCCAACCAUACCCAACCCUCCCCCCUGGUACCGGGCGGAGGAUUCAUUCCUAUCGGGGAC

ACCUUUGGCUCUUCCGAGACGCUGGGACUCACGAUGGCCUGUUGGUCAAUCAGACCGAACU

GUUUGUGCCCAGCCUGAACGUAGACGGGCAGCCAAUUUUCGCAAACAUAACACUGCCCGUA

UAUACAUUGAAAGAAAGGUGUCUUCAGGUGGUACGCAGUCUGGUUAAACCAGAAAACUAUA

GGCGUCUGGAUAUCGUGCGCAGCCUCUAUGAGGACCUUGAAGACCACCCUAACGUGCAAAA

GGACCUCGAGCGGCUCACUCAGGAGCGCAUAGCUCAUCAGCGCAUGGGAGAUGAAAACCUU

UACUUUCAGGGUGGGAGCGGCGGCAGCGGCGGGAGUCAUCAUCACCAUCAUCAUCACCACU

GA-3′ UTR- 3‵ poly A tail
SEQ ID NO: 19: mRNA sequence 2 encoding protein that degrades KRAS

5′ cap-5′UTR-

AUGGAAGUGCAACUUCUCGAAUCUGGAGGGGGGUUGGUACAACCCGGCGGCAGCUU

GCGCUUGUCCUGUGCUGCCAGUGGGUUCACAUUUUCUACAUUCUCUAUGAACUGGGUUAGA

CAAGCCCCUGGCAAAGGGCUGGAAUGGGUGAGCUAUAUAAGUCGCACCUCCAAAACUAUUU
ACUAUGCAGAUAGUGUCAAGGGACGAUUUACUAUCAGCAGAGAUAACUCUAAGAACACUCU
GUAUCUCCAGAUGAAUUCCCUUCGAGCCGAAGACACCGCAGUAUAUUACUGUGCUAGAGGG
CGCUUCUUCGACUACUGGGGGCAAGGCACUCUGGUAACUGUCAGUAGCGGGGGUUCUGAGG
GCAAAAGUUCAGGAUCUGGGUCCGAGUCUAAGUCAACUGGCGGGAGCGACAUACAAAUGAC
UCAGAGCCCCAGUAGCCUGAGCGCCUCCGUUGGUGACAGAGUCACUAUUACAUGUCGGGCA
UCUCAAUCUAUUUCCUCUUAUCUUAACUGGUAUCAGCAAAAGCCAGGCGAAGCCCCCAAAC
UCCUGAUAUACUCCGCUAGUGUUCUUCAGAGUGGCGUUCCUAGUCGCUUCAGCGGAUCCGG
AAGUGGCACUGAUUUUACACUUACAAUUAGUUCCUUGCAGCCUGAAGAUUUUGCCACAUAC
UACUGUCAGCAAUCAGUCAUGAUUCCCAUGACCUUUGGGCAAGGGACCAAAGUUGAGAUCA
AAGGCUCCGGAGGAGGAGGGUCUAUGGAAAACAGAUGGCAAGUUAUGAUCGUUUGGCAAGU
AGAUCGUAUGCGAAUACGCACAUGGAAAUCACUGGUAAAGCACCACAUGUAUGUGUCAGGC
AAAGCACGAGGAUGGUUUUAUCGUCAUCACUAUGAGUCACCACAUCCCCGCAUCAGCUCUG
AGGUGCACAUACCACUCGGUGAUGCUCGUCUGGUCAUAACUACCUACUGGGGCCUCCACAC
UGGUGAAAGAGAUUGGCACCUCGGCCAAGGAGUAAGUAUAGAAUGGCGCAAAAAGCGUUAU
UCAACCCAAGUGGAUCCUGAGUUGGCAGAUCAGUUGAUCCACUUGUAUUACUUUGACUGCU
UCAGCGAUAGUGCUAUUCGGAAGGCCCUCCUCGGGCACAUUGUAAGCCCACGGUGCGAGUA
UCAAGCUGGUCAUAACAAGGUUGGUAGCCUCCAGUACUUGGCUUUGGCUGCACUUAUAACA
CCCAAAAAGAUAAAACCUCCACUCCCCUCAGUGACUAAACUCACUGAGGAUCGUUGGAACA
AACCCCAGAAAACCAAGGGGCACAGGGGUUCACACACCAUGAAUGGCCACGAGAAUUUGUA
UUUCCAAGGGGGAUCCGGGGGCAGUGGAGGGUCUCACCAUCAUCACCACCACCACCAUUGA
-3′ UTR-3′ poly A tail

In one example of the present invention, siRNA was used to inhibit the expression of KRAS. The siRNA may be may have the nucleotide sequences of SEQ ID NO: 20 and SEQ ID NO: 21, without being limited thereto.
SEQ ID NO: 20 and SEQ ID NO: 21: siRNA sequences that inhibit expression of KRAS
SEQ ID NO: 20 (sense): CAGCUAAUUCAGAAUCAUU
SEQ ID NO: 21 (AntiSense): AAUGAUUCUGAAUUAGCUG

In the present invention, the siRNA may be naive or chemically modified siRNA, or be chemically bound to the sulfhydryl group of cysteine in the cell-penetrating and target-recognizing peptides by a chemical crosslinking agent using siRNA substituted with a sulfhydryl group or an amine group at the 5' end.

In the present invention, the mRNA may encode a target protein, recombinant protein, or viral antigen, expression of which is to be increased. The mRNA may be naive mRNA with a 3' cap and a 5' poly A tail, or may be bound to a sulfhydryl group of cysteine in the cell-penetrating and cell-recognizing peptide using a chemical cross-linking agent after binding the sulfhydryl group or amine group to the 5' end.

In the present invention, after producing Structural Formula 1, an oligonucleotide may be bound to Structural Formula 1, specifically, A in Structural Formula 1, and allowed to stand for a predetermined period of time, so that nanoparticles may be formed by self-assembly.

The nanoparticle may have a size of 10 to 200 nm, without being limited thereto.

In the present invention, the molecular weight ratio between the siRNA or mRNA and the conjugate may be 1:1 to 1:100.

In the present invention, the siRNA and mRNA may be used as anticancer drugs, without being limited thereto.

In still another aspect, the present invention relates to a composition for intracellular delivery of an oligonucleotide comprising a nanoparticle in which the peptide-based conjugate and the oligonucleotide are bound to each other.

In yet another aspect, the present invention relates to a pharmaceutical composition for preventing or treating a disease comprising a nanoparticle in which the peptide-based conjugate and an oligonucleotide are bound to each other.

In the present invention, the disease may be cancer, an inflammatory disease, or a viral infection, without being limited thereto.

The cancer may be squamous cell carcinoma, basal cell carcinoma, adenocarcinoma, hepatocellular carcinoma, renal cell carcinoma, bladder cancer, bowel cancer, breast cancer, cervical cancer, uterine cancer, colon cancer, esophageal cancer, head cancer, kidney cancer, liver cancer, lung cancer, ovarian cancer, pancreatic cancer, prostate cancer, gastric cancer, leukemia, benign and malignant lymphomas, especially Burkitt's lymphoma and non-Hodgkin's lymphoma, benign and malignant melanoma, myeloproliferative diseases, sarcoma including Ewing's sarcoma, angiosarcoma, Kaposi's sarcoma, liposarcoma, myoma, neuroepithelial sarcoma, synovial sarcoma, neurosarcoma, astrocytoma, oligodendroglioma, encephalocytoma, glioblastoma, neuroblastoma, gangliocytoma, gangliocytoma, hydroblastoma, pineal tumors, meningioma, meningeal sarcoma, neurofibroma and schwannoma, testicular cancer, thyroid cancer, carcinosarcoma, Hodgkin's, Wilms tumor, or teratocarcinoma.

The inflammatory diseases may be, but is not limited to, atopy, psoriasis, arthritis, dermatitis, allergies, osteoarthritis, rhinitis, otitis media, pharyngitis, tonsillitis, periodontitis, gingivitis, inflammatory eye disease, cystitis, nephritis, rheumatoid arthritis, spondylitis, inflammatory bowel disease, hepatitis, sepsis, alcoholic liver disease, non-alcoholic fatty liver disease, epilepsy, or intervertebral disc degeneration.

The viral infections may be, but is not limited to, a cold, flu (influenza), infectious mononucleosis, cytomegalovirus infection, measles, polio, yellow fever, dengue fever, hepatitis B, hepatitis C, AIDS, or COVID-19.

In the present invention, the pharmaceutical composition may be for the prevention or treatment of, in addition to the above-described cancer, inflammatory disease or viral infection, asthma, autoimmune disease, multiple sclerosis, ciliary disease, cleft palate, diabetes, heart disease, hypertension, mental retardation, mood disorders, obesity, refractive error, infertility, Angelman syndrome, Canavan disease, chronic digestive disorders, Charcot-Marie-Tooth (CMT) disease, cystic fibrosis, Duchenne muscular dystrophy, hemochromatosis, hemophilia, Klinefelter syndrome, neurofibromatosis, phenylketonuria, autosomal dominant polycystic neoplasm (PKD1 or PKD2), Prader-Willi syndrome, sickle cell anemia, Tay-Sachs disease, Turner syndrome, or the like, without being limited thereto.

In the present invention, the term "prevention" means any action that suppresses or delays a disease by administering the composition, and the term "treatment" means any action that ameliorates or completely cures the symptoms of a disease by administering the composition.

In the present invention, the pharmaceutical composition may be prepared in any one dosage form selected from the group consisting of injections, preparations for oral administration, patches, solutions, capsules, granules, tablets, powders, sprays, ointments, gels, mucosal preparations, and suppositories, without being limited thereto. These dosage forms may be prepared by conventional methods used for formulation in the art or by a method disclosed in Remington's Pharmaceutical Science (latest edition), Mack Publishing Company, Easton PA, and the pharmaceutical composition may be prepared in various dosage forms depending on each disease or ingredient. However, the description is illustrative and dosage forms to which the present invention is applicable are not limited to those described above.

In the present invention, the pharmaceutical composition may further comprise an acceptable excipient, and the excipient may be, for example, a carrier. A pharmaceutically acceptable carrier may be saline, sterile water, Ringer's solution, buffered saline, dextrose solution, maltodextrin solution, glycerol, ethanol, or a mixture of one or more of these components, and, if necessary, the pharmaceutical composition may further comprise other conventional additives such as antioxidants, buffers, or bacteriostatic agents. In addition, a diluent, a dispersant, a surfactant, a binder, and a lubricant may be additionally added to prepare a pill, capsule, granule, or tablet or to prepare an injectable dosage form such as an aqueous solution, suspension, or emulsion. However, the above description is illustrative and the excipient or carrier usable in the present invention is not limited to those described above.

In still yet another aspect, the present invention relates to a method for preventing or treating a disease comprising administering the nanoparticle.

In a further aspect, the present invention relates to the use of the nanoparticle for the prevention or treatment of a disease.

In another further aspect, the present invention relates to the use of the nanoparticle for the manufacture of a medicament for the prevention or treatment of a disease.

In the present invention, the method and use comprise the nanoparticle according to the present invention, and relate to the pharmaceutical composition comprising the nanoparticle, and the description of contents overlapping with the above-described nanoparticle and pharmaceutical composition are omitted.

Hereinafter, the present invention will be described in more detail by way of examples. These examples are only intended to illustrate the present invention, and it will be apparent to those skilled in the art that the scope of the present invention is not to be construed as being limited by these examples.

### Example 1: Synthesis of Peptides and Peptide-Lipid Conjugate

Amino acids and reagents required for synthesis were purchased from GL Biochem and Sigma-Aldrich. A peptide was synthesized from the C-terminus by the Fmoc solid-state chemical synthesis method using a peptide synthesizer. That is, a peptide was synthesized using a rink resin to which 2-chlorotrityl chloride was bound as a blocking group. 50 mg of the rink resin was placed in the peptide synthesizer, the resin was swelled with DMF, and then a 20% piperidine/DMF solution was used to remove the Fmoc-group. According to the sequence from the C-terminus, a 0.5M amino acid solution (solvent: dimethylformamide, DMF), 1.0M DIPEA (solvent: dimethylformamide & n-methylpyrrolidone, DMF&NMP), and 0.5M HBTU (solvent: dimethylformamide, DMF) were added to the resin in amounts of 5, 10, and 5 equivalents, respectively, and allowed to react under a nitrogen atmosphere for 1 to 2 hours. After completion of each of the deprotection and coupling steps, washing was performed twice with DMF and isopropanol. Even after coupling the last amino acid, deprotection was performed to remove the Fmoc-group. The reaction was performed using a Kaiser test solution until a negative result was obtained. After completion of the reaction, the resin was washed with DMF and MeOH and dried in a vacuum oven. An acetic acid (AcOH) cleavage cocktail was added to the resin in an amount of 15 ml per g of the resin, followed by shaking for 1 hour, and then a cocktail containing the resin and peptide dissolved therein was separated by filtration. After removing a portion of the filtered solution using a rotary evaporator, cold ether was added or an excess of cold ether was directly added to the AcOH cocktail solution containing the peptide dissolved therein to crystallize the peptide into a solid phase, which was then separated by centrifugation. At this time, the AcOH cocktail was completely removed by washing several times with ether and a centrifugation process. The peptide thus obtained was dried in a vacuum oven at room temperature. After drying, DIPEA (5 equivalents), EDC (5 equivalents), DMAP (1 equivalent), and pentadecanedioic acid (5 equivalents) were added thereto and synthesis was performed in the presence of DCM. At this time, instead of the lipid pentadecanedioic acid, a lipid such as hexadecanedioic acid, heptadecanedioic acid, octadecanedioic acid, or nonadecanedioic acid, in which carboxyl groups (-COOH) exist on both sides, may be used.

After the synthesis was completed, the solution was washed several times with water and DCM using a separatory funnel to obtain the organic layer. The peptide-lipid-peptide thus obtained was dried using a rotary evaporator, and trifluoroacetic acid (TFA) cleavage cocktail was added to the resin in an amount of 20 ml per g of the resin, followed by shaking for 3 hours to remove the remaining protecting groups from each amino acid of the peptide. After removing the solution using a rotary evaporator, cold ether was added or an excess of cold ether was directly added to the TFA cocktail solution containing the peptide-lipid conjugate dissolved therein to crystallize the peptide-lipid conjugate into a solid phase, which was then separated by centrifugation. At this time, the TFA cocktail was completely removed by washing several times with ether and a centrifugation process. The peptide-lipid-peptide thus obtained was dissolved in distilled water, lyophilized, and then separated and purified by high-performance liquid chromatography (Shimadzu, Japan). Analysis was performed using a C₁₈ column with a diameter of 4.6 mm by flowing 0.1% TFA/H₂O and 0.092% TFA/acetonitrile with a change of 0 to 60% at a flow rate of 1 ml/min for 30 minutes. At this time, the wavelength of the ultraviolet detector was 220 nm. Purification was performed using a column with a diameter of 2.2 cm at a flow rate of 20 ml/min under the same solvent and detection wavelength conditions. The molecular weight of the purified peptide was determined by mass spectrometry.

### Example 2: Production of Nanoparticles by Binding of mRNA to Peptide-Lipid-Peptide Conjugate

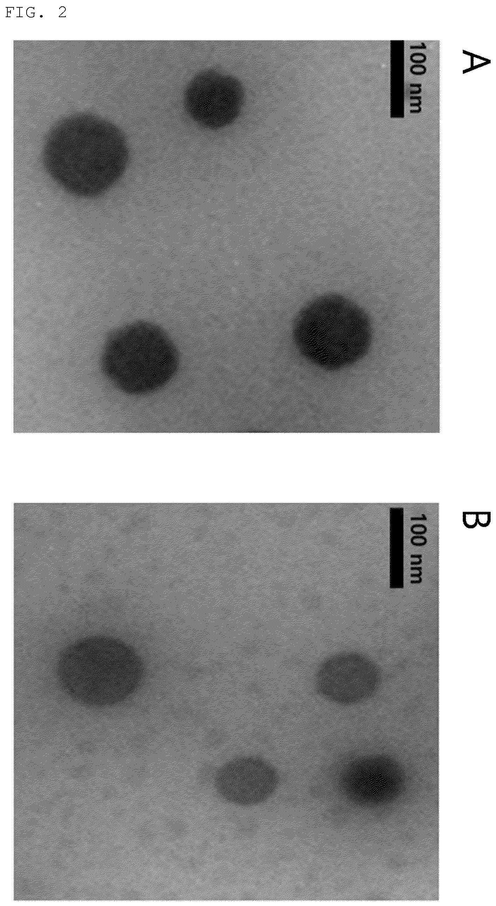

Nanoparticles were produced by binding each of mRNA (SEQ ID NO: 18 or SEQ ID NO: 19) of a gene encoding a protein that degrades mutant KRAS and siRNA (SEQ ID NO: 20 and SEQ ID NO: 21) that inhibits KRAS expression to the peptide-lipid-peptide conjugate produced in Example 1. 5 mg of the peptide-lipid-peptide conjugate was mixed with and dissolved in 1 ml of nuclease-free water (Invitrogen, AM9938). mRNA or siRNA was mixed with the peptide-lipid-peptide conjugate at a weight ratio of 1:10 and allowed to react at room temperature for 30 minutes. The formed nanoparticles were observed using a transmission electron microscope (TEM, JEM-1230, JEOL) (FIG. 2). Either mRNA or siRNA formed particles with a diameter of 100 to 120 nm.

### Example 3: Confirmation of Reduction of KRAS by mRNA Delivered into Cells

In order to confirm the intracellular expression of KRAS degradation protein, cells were treated with the nanoparticles produced by binding of mRNA encoding KRAS degradation protein to the peptide-lipid-peptide conjugate prepared in Example 2.

H358 cells (ATCC, CRL-5807) were seeded in a 6-well plate at a density of 70%. After 16 hours, the cells were starved with serum-free RPMI-1640 overnight. The medium was treated with a medium containing the nanoparticles formed by mRNA and the peptide-lipid-peptide conjugate, and the cells were harvested at hourly intervals. Active KRAS was precipitated by immunoprecipitation using the active Ras detection Kit (CST, #8821). The protein was lysed using lysis/binding/wash buffer containing protease inhibitors and phosphatase inhibitors. Protein lysis was performed using a lysis/binding/wash buffer containing a protease inhibitor and a phosphatase inhibitor. Protein quantification was performed by BCA protein assay (23227, Thermo Scientific), and the expression levels of His tag, GAPDH, and GST and active KRAS protein separated by immunoprecipitation were analyzed by Western blot analysis. For Western blot analysis, equal amounts of samples were loaded onto a 12% SDS PAGE gel along with a size marker, electrophoresed for about 2 hours, and then transferred to a nitrocellulose membrane. The transferred membrane was blocked with 5% skim milk for 30 minutes, and incubated with primary antibodies (GST, Abcam, ab19256; KRAS, Abcam, ab275876; GAPDH, Santa Cruz, sc-32233; His tag, Abcam, ab18184) at a ratio of 1:1,000 overnight. Thereafter, the membrane was washed with TBST containing 0.1% Tween-20, and incubated with HRP-conjugated secondary antibodies (anti-rabbit, A90-116P, BETHYL Lab.; anti-mouse, A120-101P, BETHYL Lab.), and then chemiluminescence was detected with an ECL substrate.

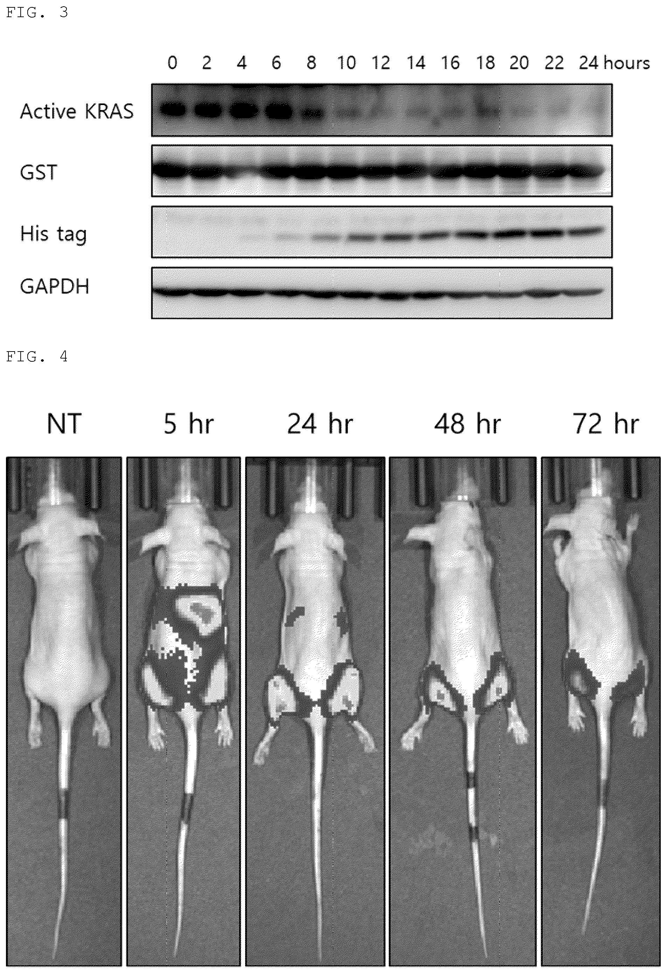

As a result, as shown in FIG. 3, it was confirmed that the KRAS degradation protein was expressed by the mRNA delivered into the cells 4 hours after nanoparticle treatment, and that the expression of active KRAS was inhibited by the expression of the KRAS degradation protein.

### Example 4: Evaluation of In Vivo Expression

In order to evaluate the *in vivo* delivery and expression of mRNA, a living body was treated with the nanoparticles formed by mRNA and the peptide-lipid-peptide conjugate.

The nanoparticles prepared as in Example 2 using firefly luciferase mRNA (FLuc mRNA, Messenger Bio, FLUC500P) were injected into the thigh muscle of C57BL/6 mice. The expression of firefly luciferase was evaluated using an IVIS instrument (PerkinElmer, IVIS SPECTRUM) at 5, 24, 48, and 72 hours. A substrate solution (VivoGlo^{™} Luciferin, In Vivo Grade, Promega, P1041) was intravenously injected into mice at 1.5 mg/kg, and the mice were evaluated by IVIS after 5 minutes.

As a result, as shown in FIG. 4, it could be confirmed that firefly luciferase was expressed *in vivo* from 5 hours to 72 hours.

### Industrial Applicability

The nanoparticles comprising the peptide-based conjugate to which an oligonucleotide bound according to the present invention are capable of effectively delivering a therapeutic oligonucleotide into cells, and may be used as a therapeutic agent by decreasing or increasing the expression of a target protein by the oligonucleotide.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

### Sequence Listing Free Text

An electronic file is attached.

## Claims

1. A peptide-based conjugate having Structural Formula 1 below as a basic unit:
[Structural Formula 1] A-B-D-E
wherein A is an RNA-binding peptide,
B is a fatty acid having 12 to 20 carbon atoms, a cationic lipid, an amphipathic polypeptide, or an amphipathic polymer,
D is a peptide having a cell-penetrating function, and
E is a peptide that recognizes a target cell surface.

2. The conjugate of claim 1, wherein A is a peptide represented by any one of the amino acid sequences of SEQ ID NO: 1 to SEQ ID NO: 15.

3. The conjugate of claim 1, wherein D is a peptide selected from the group consisting of 4 to 12 arginines, 4 to 12 lysines, and 2 cysteines, or a combination thereof.

4. The conjugate of claim 1, wherein E is a peptide represented by the amino acid sequence of SEQ ID NO: 17.

5. The conjugate of claim 1, further comprising C, a peptide that facilitates endosomal escape, between B and D.

6. The conjugate of claim 5, wherein C is a peptide consisting of 4 to 12 histidines.

7. The conjugate of claim 5, wherein A-B or B-C are linked to each other by an amide bond.

8. The conjugate of claim 7, wherein the amide bond between A-B or B-C is formed by a chemical synthesis method.

9. The conjugate of claim 5, wherein C-D-E is produced by a chemical synthesis technique or a recombinant expression technique.

10. A nanoparticle in which an oligonucleotide is bound to the peptide-based conjugate of any one of claims 1 to 9.

11. The nanoparticle of claim 10, wherein the oligonucleotide is selected from the group consisting of DNA, siRNA, miRNA, and mRNA.

12. The nanoparticle of claim 10, wherein the oligonucleotide inhibits or increase expression of a target gene.

13. The nanoparticle of claim 11, wherein the siRNA is an siRNA having a nucleotide sequence complementary to a nucleotide sequence encoding a tumor-causing protein or a disease-causing protein.

14. The nanoparticle of claim 13, wherein the siRNA is naive or chemically modified siRNA, or is chemically bound to a sulfhydryl group of cysteine in cell-penetrating and target-recognizing peptides by a chemical crosslinking agent using siRNA substituted with a sulfhydryl group or an amine group at the 5' end.

15. The nanoparticle of claim 11, wherein the mRNA encodes a target protein, recombinant protein, or viral antigen, expression of which is to be increased.

16. The nanoparticle of claim 10, wherein the oligonucleotide is bound to A in Structural Formula 1, and then the nanoparticle is formed by self-assembly of the peptide-based conjugate.

17. The nanoparticle of claim 16, which has a size of 10 to 200 nm.

18. The nanoparticle of claim 11, wherein oligonucleotide is siRNA or mRNA, wherein a molecular weight ratio between the siRNA or mRNA and the conjugate is 1:1 to 1:100.

19. A composition for intracellular delivery of an oligonucleotide comprising the nanoparticle of claim 10.

20. A pharmaceutical composition for preventing or treating a disease comprising the nanoparticle of claim 10.

21. The pharmaceutical composition of claim 20, wherein the disease is cancer, an inflammatory disease, or a viral infection.
